Europäisches Patentamt

European Patent Office

Office européen des brevets

(11) Veröffentlichungsnummer: **0 325 975**
**A1**

## EUROPÄISCHE PATENTANMELDUNG

(21) Anmeldenummer: 89100618.1

(22) Anmeldetag: 14.01.89

(51) Int. Cl.4: **C07C 139/14 , C07C 143/24**

(30) Priorität: 23.01.88 DE 3801942

(43) Veröffentlichungstag der Anmeldung:
02.08.89 Patentblatt 89/31

(84) Benannte Vertragsstaaten:
CH DE FR GB IT LI

(71) Anmelder: **BASF Aktiengesellschaft**
**Carl-Bosch-Strasse 38**
**D-6700 Ludwigshafen(DE)**

(72) Erfinder: **Eichenauer, Ulrich, Dr.**
**Paul-Ehrlich-Strasse 25a**
**D-6000 Frankfurt 70(DE)**
Erfinder: **Neumann, Peter, Dr.**
**Poststrasse 28**
**D-6800 Mannheim 31(DE)**

(54) **Verfahren zur Herstellung von Alkalisalzen der Biphenyl-4,4'-disulfonsäure.**

(57) Verfahren zur Herstellung des Dilithium-, Dinatrium- oder Dikaliumsalzes der Biphenyl-4,4'-disulfonsäure durch Sulfonierung von Biphenyl und Überführung der dabei entstehenden freien Biphenyl-4,4'-disulfonsäure in die Salzform, wobei man das nach der Sulfonierung anfallende Reaktionsgemisch mit Wasser verdünnt, die Biphenyl-4,4'-disulfonsäure durch Zugabe eines sekundären oder tertiären Amins, das mindestens 12 Kohlenstoffatome aufweist, in ihr Aminsalz überführt, dieses von der wäßrigen Phase abtrennt und es dann mit Lithium-, Natrium- oder Kaliumhydroxid behandelt.

EP 0 325 975 A1

## Verfahren zur Herstellung von Alkalisalzen der Biphenyl-4,4'-disulfonsäure

Die vorliegende Erfindung betrifft ein neues Verfahren zur Herstellung des Dilithium-, Dinatrium- oder Dikaliumsalzes der Biphenyl-4,4'-disulfonsäure durch Sulfonierung von Biphenyl, Verdünnung des anfallenden Reaktionsgemisches mit Wasser, Zugabe eines sekundären oder tertiären Amins, Abtrennung des gebildeten Aminsalzes der Biphenyl-4,4'-disulfonsäure von der wäßrigen Phase und Behandlung dieses Salzes mit Lithium-, Natrium- oder Kaliumhydroxid.

Die Herstellung von Alkalisalzen der Biphenyl-4,4'-disulfonsäure aus der im Sulfoniergemisch enthaltenen freien Disulfonsäure ist bekannt und wird entweder durch Umsetzung der freien Säure mit Alkalihydroxiden oder durch Aussalzen mittels Alkalihalogeniden bewerkstelligt (Ann. Chem. Band 132, S. 209, 1884; Helv. Chim. Acta Band 14, S. 751, 1931; US-A- 4 382 896; DE-A- 3 226 391).

Die genannten Methoden sind jedoch für technische Verfahren aus mehreren Gründen ungeeignet. So werden bei der Umsetzung mit Alkalihydroxiden große Mengen dieser Agentien benötigt. Die dabei gleichzeitig gebildeten Alkalisulfate können Folgereaktionen, wie die Alkalischmelze zu 4,4'-Dihydroxibiphenyl, behindern. Die durch Aussalzen gewonnenen Alkalisulfonate enthalten ebenfalls Alkalisulfate und/oder Alkalichloride, wobei auch die Alkalichloride aufgrund ihres korrosiven Verhaltens gegenüber bestimmten Werkstoffen, Folgereaktionen behindern können.

Um die genannten Biphenyl-4,4'-disulfonate in reiner Form zu erhalten, sind daher zusätzlich aufwendige Reinigungsschritte erforderlich.

Aufgabe der vorliegenden Erfindung war es nun, ein neues Verfahren zur Herstellung von Alkalimetallsalzen der Biphenyl-4,4'-disulfonsäure bereitzustellen, mittels dessen diese Salze in einfacher Weise in praktisch chlorid- und sulfatfreier Form erhalten werden und bei dem der Anfall an Fremdsalz möglichst gering gehalten wird.

Es wurde gefunden, daß die Herstellung des Dilithium-, Dinatrium- oder Dikaliumsalzes der Biphenyl-4,4'-disulfonsäure durch Sulfonierung von Biphenyl und Überführung der dabei entstehenden freien Biphenyl-4,4'-disulfonsäure in die Salzform vorteilhaft gelingt, wenn man das nach der Sulfonierung anfallende Reaktionsgemisch mit Wasser verdünnt, die Biphenyl-4,4'-disulfonsäure durch Zugabe eines sekundären oder tertiären Amins, das mindestens 12 Kohlenstoffatome aufweist, in ihr Aminsalz überführt, dieses von der wäßrigen Phase abtrennt und es dann mit Lithium-, Natrium- oder Kaliumhydroxid behandelt.

Die im erfindungsgemäßen Verfahren verwendeten sekundären oder tertiären Amine sollen mindestens 12 Kohlenstoffatome aufweisen. Bevorzugt sind solche Amine, die 16 bis 24 Kohlenstoffatome besitzen.

Als sekundäre oder tertiäre Amine kommen die entsprechenden Alkylamine, Cycloalkylamine oder auch gemischte N-Alkyl-N-cycloalkylamine in Betracht. Die Alkylgruppen sind dabei jeweils geradkettig oder verzweigt und können jeweils durch Phenyl substituiert sein.

Geeignete sekundäre Amine sind z.B. Dibenzylamin, Dihexylamin, Dicyclohexylamin, Bis(2-ethylhexyl)amin, N-Ethyl-N-decylamin oder N-Cyclohexyl-N-decylamin.

Geeignete tertiäre Amine sind z.B. Tribenzylamin, Tributylamin, Triisobutylamin, Tripentylamin, Trihexylamin, Tris(2-ethylhexyl)amin, N,N-Dimethyl-N-decylamin, N,N-Diethyl-N-decylamin, N,N-Dipropyl-N-cyclohexylamin, N-Methyl-N,N-dicyclohexylamin, N,N-Dipropyl-N-benzylamin oder N-Methyl-N,N-dibenzylamin.

Besonders hervorzuheben ist die Verwendung von Bis(2-ethylhexyl)amin oder Tris(2-ethylhexyl)amin.

Die Sulfonierung von Biphenyl zu Biphenyl-4,4'-disulfonsäure ist an sich bekannt. Als Sulfonierungsmittel kommen die üblichen Reagenzien, z.B. Schwefelsäure, Oleum oder Chlorsulfonsäure in Betracht. Die Umsetzung von Biphenyl mit dem Sulfonierungsmittel erfolgt im allgemeinen bei einer Temperatur von 80 bis 160 °C, wobei das Molverhältnis Biphenyl:Sulfonierungsmittel üblicherweise 1:4 bis 1:20 beträgt. Es ist auch möglich, die Sulfonierung in einer Schmelze aus Biphenyl vorzunehmen.

Erfindungsgemäß wird das bei der Sulfonierung anfallende Reaktionsgemisch mit Wasser verdünnt. Vorzugsweise verdünnt man dabei das Reaktionsgemisch bis zu einer Schwefelsäurekonzentration von 2 bis 30 Gew.%, insbesondere 10 bis 20 Gew.%, jeweils bezogen auf die Mischung.

Dem so verdünnten Sulfonierungsgemisch wird anschließend das sekundäre oder tertiäre Amin zugegeben. Die Zugabe erfolgt bei einer Temperatur von 20 bis 9 °C, wobei das Molverhältnis Biphenyl-4,4'-disulfonsäure:Amin in der Regel 1:1,9 bis 1:12, vorzugsweise 1: 1,9 bis 1: 2,5 beträgt.

Das Amin wird dabei entweder unverdünnt oder in Lösung zugegeben. Als Lösungsmittel für das Amin kommen inerte organische Lösungsmittel in Betracht, die in Wasser nicht oder nur wenig löslich sind. Solche Lösungsmittel sind z.B. Aromaten, wie Toluol, Xylol, Chlorbenzol oder Dichlorbenzol; halogenierte Alkane oder Alkene, wie Dichlormethan, Trichlormethan, Tetrachlormethan, 1,1,1-Trichlorethan, 1,1,2-Trichlorethan, 1-Fluor-1,2-dichlorethan, 1,1-Dichlorethan, 1,2-Dichlorethan, 1,1,2-Trichlorethan oder Heptafluorpropan; Ester, wie Essigsäureethylester, Essigsäurebutylester oder Essigsäureisobutylester; Ether, wie Diethylether oder Methyl-tert-butylether; oder Alkohole, wie Isobutanol, Pentanol oder Isopentanol.

Das Gewichtsverhältnis Amin:Lösungsmittel beträgt zweckmäßig 1 : 1 bis 1 : 10, vorzugsweise 1 : 1 bis 1 : 2.

Nach der Aminzugabe bilden sich eine wäßrige, Schwefelsäure enthaltende Phase und eine das Aminsalz der Biphenyl-4,4'-disulfonsäure enthaltende Phase aus. Die das Aminsalz enthaltende Phase fällt dabei in flüssiger oder fester Form an. Die Gewinnung der Aminsalz-Phase erfolgt dabei nach an sich bekannten Methoden. Für den Fall, daß das Aminsalz in flüssiger Phase vorliegt, kann die wäßrige, Schwefelsäure enthaltende Phase, beispielsweise durch einfache Flüssig-Flüssig-Phasentrennung, entfernt werden. Für den Fall, daß das Aminsalz in fester Phase anfällt, kann es von der wäßrigen Phase, z.B. durch Filtration, getrennt werden.

Die so isolierte Aminsalzphase, die gegebenenfalls noch inertes organisches Lösungsmittel enthält, wird anschließend mit Lithium-, Natrium- oder Kaliumhydroxid behandelt, wobei freies Amin und das Dilithium-, Dinatrium- oder Dikaliumsalz der Biphenyl-4,4'-disulfonsäure gebildet wird. Das Alkalihydroxid wird dabei in fester Form oder vorzugsweise als wäßrige Lösung zugegeben, wobei der Gehalt an Alkalihydroxid im allgemeinen dann 10 bis 50 Gew.% beträgt. Bezogen auf 1 Mol Biphenyl-4,4'-disulfonsäure werden dabei 1,9 bis 5 Mol, vorzugsweise 1,9 bis 2,5 Mol Lithium-, Natrium- oder Kaliumhydroxid verwendet.

Je nach Wassergehalt fällt das Dilithium-, Dinatrium- oder Dikaliumsalz der Biphenyl-4,4'-disulfonsäure dabei in fester Form, als Suspension oder als wäßrige Lösung an. Das freigesetzte Amin liegt als flüssige organische Phase vor, die gegebenenfalls noch inertes organisches Lösungsmittel enthält.

Die Trennung von Amin und Disulfonat erfolgt bei einer Temperatur von 20 bis 95 °C, wobei auch hier die üblichen, an sich bekannten Methoden angewandt werden. So kann die Amin-Phase beispielsweise durch Flüssig-Flüssig-Phasentrennung, durch Extraktion oder durch Filtration entfernt werden. Für den Fall, daß das Alkalidisulfonat als Suspension anfällt, kann beispielsweise der in fester Form vorliegende Anteil durch Filtration und der als wäßrige Lösung vorliegende Anteil durch anschließende Flüssig-Flüssig-Phasentrennung gewonnen werden. Das freie Amin, das gegebenenfalls noch inertes organisches Lösungsmittel enthält, kann ohne nennenswerte Verluste in das Verfahren rückgeführt werden.

Mittels des erfindungsgemäßen Verfahrens werden die genannten Alkalidisulfonate in hoher Reinheit und in guten Ausbeuten erhalten. Wie oben bereits ausgeführt, sind sie praktisch frei von Chlorid- oder Sulfationen. Weiterhin ist die hohe Selektivität bei der Trennung Sulfonsäure-Schwefelsäure hervorzuheben. Schließlich zeichnet sich das neue Verfahren durch leichte technische Realisierbarkeit aus, wobei das Zielprodukt, je nach Wahl der Reaktionsbedingungen, in kristalliner Form oder als wäßrige Lösung anfällt.

Biphenyl-4,4'-disulfonsäure und insbesondere ihre Alkalisalze sind wertvolle Zwischenprodukte für die Herstellung von 4,4'-Dihydroxybiphenyl, das als Monomer für thermoplastische flüssigkristalline Polymere sowie als Bestandteil von photographischen Entwicklern zur Anwendung kommt.

Die folgenden Beispiele sollen die Erfindung näher erläutern.

Beispiel 1

77 g (0,5 Mol) Biphenyl wurden in 250 g (2,5 Mol) konz. Schwefelsäure suspendiert und 4 Stunden bei 145 °C gerührt. Man gab das Reaktionsgemisch dann auf 2 400 g Eiswasser und versetzte bei 80 °C mit 353 g (1 Mol) Tris(2-ethylhexyl)amin. Die wäßrige Phase wurde abgetrennt (Sulfonsäuregehalt < 0,5% laut potentiometrischer Titration) und die organische Phase bei 80 °C mit 123 g (1,1 Mol) 50 gew.%iger Kalilauge und 800 ml Wasser versetzt. Man erhielt nach Phasentrennung 329 g (93%) Tris(2-ethylhexyl)amin und eine Lösung von 0,47 Mol (94%) des Dikaliumsalzes der Biphenyl-4,4'-disulfonsäure in Wasser.

Beispiel 2

Es wurde analog Beispiel 1 vorgegangen. Nach Abtrennung der wäßrigen Phase wurde die organische Phase mit 80 g 50 gew.%iger Natronlauge versetzt

Danach saugte man bei 90 °C vom entstandenen Niederschlag ab. Im Filtrat wurde die organische Phase abgetrennt, wobei man Tris(2-ethylhexyl)amin (312 g, 88%) zurückerhielt. Der Filtrationsrückstand wurde mit Essigsäureethylester gewaschen und getrocknet. Man erhielt 161 g (90%) analysenreines Dinatriumsalz der Biphenyl-4,4'-disulfonsäure.

Beispiel 3

Es wurde analog Beispiel 1 vorgegangen. Nach Verdünnung des Sulfonierungsgemisches mit Wasser gab man bei 25 °C 185 g (1 Mol) Tributylamin, gelöst in 150 ml Isobutanol, zu und trennte die wäßrige Phase ab. Die organische Phase versetzte man mit 80 g (1 Mol) 50 gew.%iger Natronlauge. Der entstandene Niederschlag wurde abgesaugt und getrocknet. Man erhielt 129,3 g (72%) analysenreines Dinatriumsalz der Biphenyl-4,4'-disulfonsäure.

**Ansprüche**

1. Verfahren zur Herstellung des Dilithium-, Dinatrium- oder Dikaliumsalzes der Biphenyl-4,4'-disulfonsäure durch Sulfonierung von Biphenyl und Überführung der dabei entstehenden freien Biphenyl-4,4'-disulfonsäure in die Salzform, dadurch gekennzeichnet, daß man das nach der Sulfonierung anfallende Reaktionsgemisch mit Wasser verdünnt, die Biphenyl-4,4'-disulfonsäure durch Zugabe eines sekundären oder tertiären Amins, das mindestens 12 Kohlenstoffatome aufweist, in ihr Aminsalz überführt, dieses von der wäßrigen Phase abtrennt und es dann mit Lithium-, Natrium- oder Kaliumhydroxid behandelt.

2. Verfahren gemäß Anspruch 1, dadurch gekennzeichnet, daß man das nach der Sulfonierung anfallende Reaktionsgemisch mit Wasser bis zu einer Schwefelsäurekonzentration von 2 bis 20 Gew.%, bezogen auf die Mischung, verdünnt.

3. Verfahren gemäß Anspruch 1, dadurch gekennzeichnet, daß man die Aminzugabe bei einer Temperatur von 20 bis 95 °C vornimmt.

4. Verfahren gemäß Anspruch 1, dadurch gekennzeichnet, daß man das Amin zusammen mit einem in Wasser nicht oder nur wenig löslichen organischen Lösungsmittel zugibt.

**Europäisches Patentamt**

**EUROPÄISCHER RECHERCHENBERICHT**

Nummer der Anmeldung

| EINSCHLÄGIGE DOKUMENTE | | | EP 89100618.1 |
|---|---|---|---|
| **Kategorie** | **Kennzeichnung des Dokuments mit Angabe, soweit erforderlich, der maßgeblichen Teile** | **Betrifft Anspruch** | **KLASSIFIKATION DER ANMELDUNG (Int. Cl.4)** |
| X | PATENT ABSTRACTS OF JAPAN, unexamined applications, C Sektion, Band 1, Nr. 53, 23. Mai 1977 THE PATENT OFFICE JAPANESE GOVERNMENT Seite 323 C 77 <br><br> * Kokai-Nr. 52-12 145 (SUMITOMO KAGAKU) * <br><br> -- | 1 | C 07 C 139/14 <br> C 07 C 143/24 |
| X | EP - A2 - 0 041 134 (BASF) <br> * Zusammenfassung * <br><br> -- | 1,2 | |
| X | EP - A2 - 0 089 653 (HOECHST) <br> * Zusammenfassung * <br><br> ---- | 1 | |
| | | | **RECHERCHIERTE SACHGEBIETE (Int. Cl.4)** |
| | | | C 07 C 139/00 <br> C 07 C 143/00 |
| | Der vorliegende Recherchenbericht wurde für alle Patentansprüche erstellt | | |

| Recherchenort | Abschlußdatum der Recherche | Prüfer |
|---|---|---|
| WIEN | 22-03-1989 | REIF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82